# EUROPEAN PATENT APPLICATION

(11) **EP 4 512 404 A1**
(43) Date of publication of application: **26.02.2025**
(21) Application number: 23791945.1
(22) Date of filing: 21.04.2023
(51) Int. Cl.: A61K 31/558, A61K 9/08, A61K 47/02, A61K 47/10, A61K 47/12, A61K 47/18, A61K 47/26, A61P 27/02, A61P 27/06

(54) **COMPOSITION FOR OPHTHALMIC USE CONTAINING SEPETAPROST**

(30) Priority: 22.04.2022 JP 2022071175
(71) Applicant: Santen Pharmaceutical Co., Ltd., Kita-ku Osaka-shi Osaka 530-8552 (JP); ONO Pharmaceutical Co., Ltd., Osaka-shi, Osaka 541-8526 (JP)
(72) Inventor: ENDO Yoko, Ikoma-shi, Nara 630-0101 (JP); NAKAYAMA Sachie, Ikoma-shi, Nara 630-0101 (JP); MASAKI Kenji, Ikoma-shi, Nara 630-0101 (JP); YOSHII Kenta, Ikoma-shi, Nara 630-0101 (JP); MAEIE Tadahiro, Osaka-shi, Osaka 541-8564 (JP); TANIKAWA Seiichi, Mishima-gun, Osaka 618-8585 (JP)
(74) Representative: Grünecker Patent- und Rechtsanwälte PartG mbB
(86) International application number: PCT/JP2023/015885
(87) International publication number: WO 2023/204297

(57) **Abstract**

The purpose of the present invention is to discover a method for stabilizing a composition for ophthalmic use containing sepetaprost, more specifically, a method that facilitates retention of sepetaprost stability at high temperatures, and further, a method that facilitates the retention of preservative effectiveness of this composition for ophthalmic use. The present invention is a composition for ophthalmic use, which contains sepetaprost at a concentration of 0.0001 to 0.003% (w/v) and has a pH within a range from 5 to 8.

## Description

### TECHNICAL FIELD

The present invention relates to an ophthalmic composition comprising sepetaprost (hereinafter also referred to as an ophthalmic composition of the present invention).

### BACKGROUND ART

Glaucoma is an intractable ophthalmopathy in which an increase in intraocular pressure due to various causes of the disease damages ocular internal tissues (retinae, optic nerves, and the like) to pose a risk of blindness. As a method for treating glaucoma, therapies for lowering intraocular pressure are common and represented by pharmacotherapy, laser therapy, surgical therapy, and the like.

Drugs such as sympathomimetic drugs (non-selective stimulants such as dipivefrin; and α₂ receptor agonists such as brimonidine), sympatholytic drugs (β-blockers such as timolol, befunolol, carteolol, nipradilol, betaxolol, levobunolol, and metipranolol and α₁-blockers such as bunazosin hydrochloride), parasympathomimetic drugs (pilocarpine and the like), carbonic anhydrase inhibitors (acetazolamide and the like), prostaglandins (isopropyl unoprostone, latanoprost, travoprost, bimatoprost, and the like), and Rho kinase inhibitor (ripasudil) have been used for the pharmacotherapy.

Sepetaprost is a compound represented by formula (1): 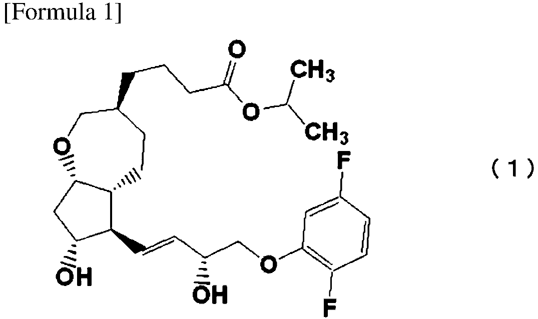 Patent Documents 1 and 2 describe sepetaprost as one of the enormous number of compounds. It is disclosed that since this compound has the action of lowering intraocular pressure powerfully and continuously, the compound can be a therapeutic agent for glaucoma (Non Patent Document 1).

In addition, uses of sepetaprost in combination with other drugs having the action of lowering intraocular pressure for obtaining the effect of lowering intraocular pressure more powerfully have been reported. For example, Patent Document 3 reports the combined administration of sepetaprost and β-blocker. Patent Document 4 reports the combined administration of sepetaprost and Rho kinase inhibitor. Patent Document 5 reports the combined administration of sepetaprost and EP2 agonist. A pharmaceutical formulation for treating or preventing glaucoma or ocular hypertension, comprising sepetaprost as the active ingredient, wherein the pharmaceutical formulation is administered to a patient on whom other therapeutic drugs for glaucoma or ocular hypertension have an insufficient effect has been reported (Patent Document 6). A pharmaceutical composition including sepetaprost for treating myopia, preventing myopia, and/or suppressing the progression of myopia has also been reported (Patent Document 7).

However, the stability or the preservative effectiveness of ophthalmic compositions containing sepetaprost has never been investigated or reported until now at all.

### PRIOR ART DOCUMENTS

### Patent Document

Patent Document 1: International Publication No. WO2011/013651
Patent Document 2: International Publication No. WO2012/102357
Patent Document 3: International Publication No. WO2019/124523
Patent Document 4: International Publication No. WO2019/124488
Patent Document 5: International Publication No. WO2019/124489
Patent Document 6: International Publication No. WO2022/034909
Patent Document 7: International Publication No. WO2021/145356

### Non Patent Document

Non Patent Document 1: Invest Ophthalmol Vis Sci. 2015 Apr;56(4):2547-52

### SUMMARY OF INVENTION

### Problems to be Solved by the Invention

The present inventors have extensively examined the ophthalmic composition containing sepetaprost and found that if the ophthalmic composition containing sepetaprost is stored at high temperatures, sepetaprost is unexpectedly destabilized. Moreover, the present inventors have found that the type of a tonicity agent contained in the ophthalmic composition unexpectedly influences the preservative effectiveness.

Accordingly, a problem to be solved by the present invention is to find a method for stabilizing the ophthalmic composition containing sepetaprost, more specifically to find a method for maintaining the stability of sepetaprost even at high temperatures (for example, at 70°C), and furthermore, a problem to be solved is to find a method enabling maintaining the preservative effectiveness of the ophthalmic composition.

### Means for Solving the Problems

The present inventors have earnestly investigated to solve the above-mentioned problem, consequently found that, in the ophthalmic composition containing sepetaprost, the adjustment of the pH to a range of 5 to 8 enables enhancing the stability of sepetaprost, further found that the blending of edetic acid or a salt thereof enables further enhancing the stability of sepetaprost, and completed the present invention.

In addition, the present inventors have found that the blending of an ionic tonicity agent into the ophthalmic composition unexpectedly enables enhancing the preservative effectiveness. Meanwhile, the present inventors have found that, in the presence of benzalkonium chloride, the blending of an ionic tonicity agent reduces the preservative effectiveness, and the blending of a nonionic tonicity agent enables maintaining the preservative effectiveness. The present inventors have found that the selection of a method for sterilizing the ophthalmic composition containing sepetaprost enables maintaining high storage stability of sepetaprost in the ophthalmic composition.

That is, the present invention provides the following.
[1] An ophthalmic composition comprising sepetaprost at a concentration of 0.0001 to 0.003% (w/v), wherein the pH of the composition is in a range of 5 to 8.
[2] The ophthalmic composition according to [1], further comprising edetic acid or a salt thereof at a concentration of more than 0.01% (w/v).
[3] The ophthalmic composition according to [2], wherein the concentration of edetic acid or a salt thereof is 0.05% (w/v) or more.
[4] The ophthalmic composition according to [2], wherein the concentration of edetic acid or a salt thereof is 0.05 to 0.5% (w/v).
[5] The ophthalmic composition according to [2], wherein the concentration of edetic acid or a salt thereof is 0.05 to 0.2% (w/v).
[6] The ophthalmic composition according to any of [1] to [5], wherein the pH is in the range of 6 to 7.
[7] The ophthalmic composition according to any of [1] to [5], wherein the pH is 6.5.
[8] The ophthalmic composition according to any of [1] to [7], further comprising a tonicity agent.
[9] The ophthalmic composition according to [8], wherein the tonicity agent is an ionic tonicity agent.
[10] The ophthalmic composition according to [9], comprising no benzalkonium chloride.
[11] The ophthalmic composition according to [9], comprising no preservative.
[12] The ophthalmic composition according to any of [9] to [11], comprising no nonionic tonicity agent.
[13] The ophthalmic composition according to [8], wherein the tonicity agent is a nonionic tonicity agent.
[14] The ophthalmic composition according to [13], further comprising benzalkonium chloride.
[15] The ophthalmic composition according to [13] or [14], comprising no ionic tonicity agent.
[16] An ophthalmic composition comprising sepetaprost at a concentration of 0.0001 to 0.003% (w/v); a nonionic tonicity agent; and benzalkonium chloride, wherein the pH of the composition is in a range of 5 to 8.
[17] The ophthalmic composition according to [16], comprising no ionic tonicity agent.
[18] An ophthalmic composition comprising sepetaprost at a concentration of 0.0001 to 0.003% (w/v); an ionic tonicity agent; and no benzalkonium chloride, wherein the pH of the composition is in a range of 5 to 8.
[19] The ophthalmic composition according to [18], comprising no preservative.
[20] An ophthalmic composition comprising:
   sepetaprost at a concentration of 0.0001 to 0.003% (w/v);
   edetic acid or a salt thereof at a concentration of more than 0.01% (w/v);
   an ionic tonicity agent;
   no nonionic tonicity agent; and
   no benzalkonium chloride,
   wherein the pH of the composition is in a range of 5 to 8.
[21] An ophthalmic composition comprising:
   sepetaprost at a concentration of 0.0001 to 0.003% (w/v);
   edetic acid or a salt thereof at a concentration of more than 0.01% (w/v);
   a nonionic tonicity agent;
   benzalkonium chloride; and
   no ionic tonicity agent,
   wherein the pH of the composition is in a range of 5 to 8.
[22] The ophthalmic composition according to any of [12] to [17], [20], and [21], wherein the nonionic tonicity agent is at least one selected from the group consisting of mannitol, glycerin, propylene glycol, polyethylene glycol, sorbitol, trehalose, maltose, and sucrose.
[23] The ophthalmic composition according to [22], wherein the nonionic tonicity agent is mannitol.
[24] The ophthalmic composition according to any of [9] to [12], [15], and [17] to [23], wherein the ionic tonicity agent is at least one selected from the group consisting of sodium chloride, potassium chloride, calcium chloride, and magnesium chloride.
[25] The ophthalmic composition according to [24], wherein the ionic tonicity agent is sodium chloride.
[26] The ophthalmic composition according to any of [1] to [25], further comprising a buffer agent.
[27] The ophthalmic composition according to any of [1] to [26], further comprising a surfactant.
[28] The ophthalmic composition according to any of [1] to [27], wherein the ophthalmic composition is a sterilized composition.
[29] The ophthalmic composition according to [28], wherein the sterilization is filtration sterilization.
[30] An ophthalmic composition comprising:
   sepetaprost at a concentration of 0.0001 to 0.003% (w/v);
   polysorbate 80 at a concentration of 0.05 to 0.3% (w/v);
   sodium citrate hydrate at a concentration of 0.1 to 0.5% (w/v);
   sodium edetate at a concentration of 0.05 to 0.5% (w/v);
   sodium chloride at a concentration of 0.5 to 1% (w/v); and
   no benzalkonium chloride,
   wherein the pH of the composition is 6 to 7.
[31] An ophthalmic composition comprising:
   sepetaprost at a concentration of 0.0001 to 0.003% (w/v);
   polysorbate 80 at a concentration of 0.05 to 0.3% (w/v);
   sodium citrate hydrate at a concentration of 0.1 to 0.5% (w/v);
   sodium edetate at a concentration of 0.05 to 0.5% (w/v);
   mannitol at a concentration of 1 to 6% (w/v); and
   benzalkonium chloride at a concentration of 0.001 to 0.0075% (w/v),
   wherein the pH of the composition is 6 to 7.
[32] An ophthalmic composition comprising:
   sepetaprost at a concentration of 0.0001 to 0.003% (w/v);
   polysorbate 80 at a concentration of 0.05 to 0.3% (w/v);
   sodium citrate hydrate at a concentration of 0.1 to 0.5% (w/v);
   sodium edetate at a concentration of 0.05 to 0.5% (w/v);
   sodium chloride at a concentration of 0.5 to 1% (w/v); and
   no benzalkonium chloride,
   wherein the pH of the composition is 6.5.
[33] An ophthalmic composition comprising:
   sepetaprost at a concentration of 0.0001 to 0.003% (w/v);
   polysorbate 80 at a concentration of 0.05 to 0.3% (w/v);
   sodium citrate hydrate at a concentration of 0.1 to 0.5% (w/v);
   sodium edetate at a concentration of 0.05 to 0.5% (w/v);
   mannitol at a concentration of 1 to 6% (w/v); and
   benzalkonium chloride at a concentration of 0.001 to 0.0075% (w/v),
   wherein the pH of the composition is 6.5.
[34] The ophthalmic composition according to any of [1] to [33], wherein the
   concentration of sepetaprost is 0.001 to 0.003% (w/v).
[35] The ophthalmic composition according to any of [1] to [33], wherein the
   concentration of sepetaprost is 0.002 to 0.003% (w/v).
[36] The ophthalmic composition according to any of [1] to [33], wherein the
   concentration of sepetaprost is 0.002% (w/v).
[37] An ophthalmic composition comprising sepetaprost at a concentration of 0.002 to 0.003% (w/v), wherein the pH of the composition is in a range of 6 to 7.
[38] An ophthalmic composition comprising sepetaprost at a concentration of 0.002% (w/v), wherein the pH of the composition is in a range of 6 to 7.
[39] The ophthalmic composition according to any of [1] to [38], wherein the ophthalmic composition is an eye drop.
[40] The ophthalmic composition according to any of [1] to [39], wherein the ophthalmic composition is contained in a unit dose eye drop container.
[41] The ophthalmic composition according to any of [1] to [39], wherein the ophthalmic composition is contained in a multi-dose eye drop container.
[42] The ophthalmic composition according to any of [1] to [41], wherein the ophthalmic composition is contained in a plastic eye drop container.
[43] The ophthalmic composition according to any of [1] to [42], wherein the ophthalmic composition is for preventing or treating glaucoma or ocular hypertension.

Moreover, the present invention also relates to the following.
A method for stabilizing sepetaprost in an ophthalmic composition, comprising adjusting a pH to a range of 5 to 8 in the ophthalmic composition comprising sepetaprost at a concentration of 0.0001 to 0.003% (w/v).
The method according to [44], wherein the pH is adjusted to the range of 6 to 7 in the ophthalmic composition comprising sepetaprost at a concentration of 0.002 to 0.003% (w/v).
The method according to [44] or [45], wherein the pH is adjusted to the range of 6 to 7 in the ophthalmic composition comprising sepetaprost at a concentration of 0.002% (w/v).
A method for stabilizing an ophthalmic composition comprising sepetaprost, comprising adding edetic acid or a salt thereof at a concentration of more than 0.01% (w/v), and adjusting a pH to a range of 5 to 8.
A method for imparting preservative effectiveness to an ophthalmic composition comprising sepetaprost, comprising adding an ionic tonicity agent. A method for imparting preservative effectiveness to an ophthalmic composition comprising sepetaprost, comprising adding a nonionic tonicity agent and benzalkonium chloride.
A method for stabilizing sepetaprost in an ophthalmic composition comprising sepetaprost, comprising sterilizing the ophthalmic composition.
The method according to [50], wherein the sterilization is filtration sterilization.
A method for suppressing a decrease in the content of sepetaprost in an ophthalmic composition comprising sepetaprost, comprising subjecting the ophthalmic composition to filtration sterilization.
A method for stabilizing sepetaprost in the ophthalmic composition according to any of [1] to [43], comprising making the ophthalmic composition contained in a plastic eye drop container.
An ophthalmic pharmaceutical product, wherein the ophthalmic composition according to any of [1] to [43] is contained in a unit dose eye drop container or a multi-dose eye drop container.
An ophthalmic pharmaceutical product, wherein the ophthalmic composition according to any of [1] to [43] is contained in a plastic eye drop container.
A method for producing an ophthalmic pharmaceutical product, comprising subjecting the ophthalmic composition according to any of [1] to [43] to sterilization.
The production method according to [56], wherein the sterilization is filtration sterilization.
The production method according to [56] or [57], further comprising making the ophthalmic composition subjected to the sterilization contained in an eye drop container.
The production method according to [56] to [58], wherein the eye drop container is a plastic eye drop container.
The production method according to [56] to [59], wherein the eye drop container is a unit dose eye drop container or a muti-dose eye drop container.

Two or more of the configurations of [1] to [60] can be combined arbitrarily.

### Advantageous Effects of Invention

The present invention enables enhancing the stability of sepetaprost in an ophthalmic composition and providing an ophthalmic composition that can maintain its stability even on exposure to high temperatures in the distribution process or the storage process. Additively, the present invention enables providing an ophthalmic composition wherein the preservative effectiveness is maintained by the use of a suitable type of tonicity agent, in addition to maintaining the stability of sepetaprost. Furthermore, the present invention enables providing an ophthalmic composition wherein high stability of sepetaprost in the ophthalmic composition is maintained by the selection of a method for sterilizing the ophthalmic composition containing sepetaprost.

### BRIEF DESCRIPTION OF DRAWINGS

[Fig. 1] Fig. 1 is a graph showing the test results in Test Example 1.
[Fig. 2] Fig. 2 is graphs showing the test results in Test Example 2.
[Fig. 3] Fig. 3 is photographs showing the test results in Test Example 4.

### DESCRIPTION OF EMBODIMENTS

Hereinafter, the present invention will be described in detail.

In the present invention, sepetaprost is a compound represented by the following formula (1): 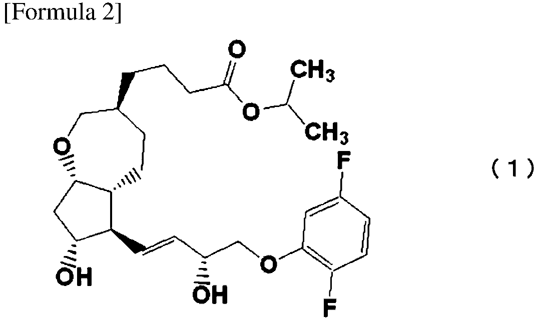 (CAS registration number: 1262873-06-2), and is also referred to as 2-propanyl 4-{(3S,5aR,6R,7R,8aS)-6-[(1E,3R)-4-(2,5-difluorophenoxy)-3-hydroxy-1-buten-1-yl]-7-hydroxyoctahydro-2H-cyclopenta[b]oxepin-3-yl}butanoate or ONO-9054.

Sepetaprost can be produced in accordance with the method described in International Publication No. WO2011/013651 (Patent Document 1), International Publication No. WO2018/003945, a common method in the art, or the like.

When sepetaprost has geometrical isomers and/or optical isomers, the scope of the present invention includes the isomers.

When sepetaprost has proton tautomerism, the present invention includes the tautomers (keto form and enol form).

When sepetaprost has crystal polymorphism and/or a crystal polymorphism group (crystal polymorphism system), the present invention also includes the crystal polymorphs and/or the crystal polymorphism group (crystal polymorphism system). The crystal polymorphism group (crystal polymorphism system) as used here means individual crystal forms and/or all thereof in various stages where crystal forms are changed according to the conditions and/or states (incidentally, this state also includes formulated state) of production, crystallization and storage of the crystals.

Sepetaprost may be in the form of a hydrate or a solvate.

In the ophthalmic composition of the present invention, the content of sepetaprost is 0.0001 to 0.003% (w/v). The lower limit of the sepetaprost content is preferably 0.0001% (w/v) or more, more preferably 0.0003% (w/v) or more, further preferably 0.001% (w/v) or more, and still further preferably 0.0015% (w/v) or more. The upper limit thereof is preferably 0.003% (w/v) or less, more preferably 0.0025% (w/v) or less, and further preferably 0.002% (w/v) or less. More specifically, the above-mentioned content can be in a range specified by combining any lower limit and any upper limit among the above-mentioned lower limits and upper limits. Examples of the range of the content include 0.0001 to 0.003% (w/v), 0.0003 to 0.003% (w/v), 0.0015% to 0.003% (w/v), 0.001 to 0.003% (w/v), 0.002 to 0.003% (w/v), 0.0001 to 0.025% (w/v), 0.0003 to 0.025% (w/v), 0.0015 to 0.025% (w/v), 0.001 to 0.025% (w/v), 0.0001 to 0.002% (w/v), 0.0003 to 0.002% (w/v), and 0.001 to 0.002% (w/v). More specifically, examples of a preferable content include 0.001% (w/v), 0.002% (w/v), and 0.003% (w/v). The content is particularly preferably 0.002% (w/v). The above-mentioned content can be a preferable content in the case where the ophthalmic composition is an eye drop. The unit "% (w/v)" as used here means the mass (g) of the active ingredient (sepetaprost) or an additive contained in 100 mL of the ophthalmic composition. For example, sepetaprost at 0.001% (w/v) means that the content of sepetaprost contained in 100 mL of the ophthalmic composition is 0.001 g. The numerical values (for example, contents, concentrations, pHs, and the like) as used herein may be accompanied with "around". In the case, the numerical values accompanied with "around" are intended to include the range of the numerical values ± 10%. For example, the phrase "around 10" shall include "9 to 11". The term "around" accompanying a numerical range indicated with the term "to" is applied to both limits of the range. Accordingly, for example, the phrase "around 10 to 20 " shall include the range "9 to 22."

When sepetaprost is in the form of a hydrate or a solvate, the content of sepetaprost may be calculated based on any of the free form, and the hydrate and the solvate of sepetaprost. The contents of other components may be calculated in the same way.

The ophthalmic composition of the present invention can contain, for example, edetic acid or a salt thereof.

Examples of edetic acid or a salt thereof to be used in the present invention include edetic acid (ethylenediaminetetraacetic acid), monosodium edetate, disodium edetate, trisodium edetate, and tetrasodium edetate. Edetic acid or a salt thereof may be a hydrate thereof. Among others, disodium edetate (hereinafter also referred to as sodium edetate) can be the most preferably used in the present invention.

In the ophthalmic composition of the present invention, the content of edetic acid or a salt thereof is more than 0.01% (w/v). The lower limit of edetic acid or a salt thereof is, for example, 0.05%(w/v) or more, preferably more than 0.05% (w/v), 0.1% (w/v) or more, and more preferably more than 0.1% (w/v). The upper limit thereof is, for example, preferably 0.5% (w/v) or less, 0.3% (w/v) or less, and more preferably 0.2% (w/v) or less. More specifically, the above-mentioned content can be in a range specified by combining any lower limit and any upper limit among the above-mentioned lower limits and upper limits. Examples of the range of the content include 0.05 to 0.5% (w/v), more than 0.05% (w/v) and 0.5% (w/v) or less, 0.05 to 0.2% (w/v), more than 0.05% (w/v) and 0.2% (w/v) or less, 0.1 to 0.3% (w/v), more than 0.1% (w/v) and 0.3% (w/v) or less, 0.1 to 0.2% (w/v), and more than 0.1% (w/v) and 0.2% (w/v) or less. More specifically, examples of a preferable content include 0.11% (w/v), 0.12% (w/v), 0.13% (w/v), 0.14% (w/v), 0.15% (w/v), 0.16% (w/v), 0.17% (w/v), 0.18% (w/v), 0.19% (w/v), and 0.2% (w/v).

The ophthalmic composition of the present invention has a pH in the range of 5 to 8. A more preferable range of pH is 5.5 to 7.5, and a further preferable range of pH is 6 to 7. More specifically, examples of a preferable pH include 6.0, 6.1, 6.2, 6.3, 6.4, 6.5, 6.6, 6.7, 6.8, 6.9, and 7.0. Among others, the pH is preferably 6.5. For example, the addition of a suitable amount of the pH adjuster described below enables adjusting the pH of the ophthalmic composition.

Additives can be used for the ophthalmic composition of the present invention as needed. As the additives, for example, a tonicity agent, a preservative, a buffer agent, a surfactant, a stabilizer, an antioxidant, a pH adjuster, a base, and the like can be added. These may be suitably used alone or in combination of two or more, and can be blended in suitable amounts.

Examples of the tonicity agent that can be blended into the ophthalmic composition of the present invention include an ionic tonicity agent and a nonionic tonicity agent.

Examples of the ionic tonicity agent include sodium chloride, potassium chloride, calcium chloride, and magnesium chloride. Among others, sodium chloride is preferable.

Examples of the nonionic tonicity agent include mannitol, glycerin, propylene glycol, polyethylene glycol, sorbitol, trehalose, maltose, and sucrose. Among others, mannitol (preferably D-mannitol) is preferable.

When the tonicity agent is blended into the ophthalmic composition of the present invention, the tonicity agents may be used alone or in combination of two or more. For example, when the ophthalmic composition of the present invention contains an ionic tonicity agent, the ophthalmic composition can contain no nonionic tonicity agent, and can also contain only an ionic tonicity agent as the tonicity agent. When the ophthalmic composition of the present invention contains a nonionic tonicity agent, the ophthalmic composition can contain no ionic tonicity agent, and can contain only a nonionic tonicity agent as the tonicity agent.

In the present invention, when the ionic tonicity agent is blended into the ophthalmic composition, the ionic tonicity agent enables imparting preservative effectiveness to the ophthalmic composition as shown in Examples, described below. The ionic tonicity agent is therefore preferably blended into the ophthalmic composition of the present invention. Although the ophthalmic composition of the present invention can contain a preservative such as benzalkonium chloride arbitrarily, it is preferable that a preservative such as benzalkonium chloride be not blended in the case where the ophthalmic composition contains an ionic tonicity agent.

When the ophthalmic composition of the present invention contains benzalkonium chloride as a preservative, the nonionic tonicity agent is preferably blended in the present invention, as described below.

When the tonicity agent is blended into the ophthalmic composition of the present invention, the content of the tonicity agents can be suitably adjusted depending on the type of the tonicity agent and the like, and is preferably 0.001 to 20% (w/v), more preferably 0.01 to 15% (w/v), and further preferably 0.1 to 10% (w/v).

More specifically, when the tonicity agent to be blended is an ionic tonicity agent, the content of the ionic tonicity agents is preferably 0.001 to 10% (w/v), more preferably 0.01 to 5% (w/v), and further preferably 0.5 to 1% (w/v). Further specific preferable examples of the content include 0.5% (w/v), 0.55% (w/v), 0.6% (w/v), 0.65% (w/v), 0.7% (w/v), 0.75% (w/v), 0.8% (w/v), 0.85% (w/v), 0.9% (w/v), 0.95% (w/v), and 1% (w/v).

More specifically, when the tonicity agent to be blended is a nonionic tonicity agent, the content of the nonionic tonicity agents is preferably 0.01 to 20% (w/v), more preferably 0.5 to 10% (w/v), and further preferably 1 to 6% (w/v). Examples of a further specific preferable content include 1% (w/v), 2% (w/v), 3% (w/v), 4% (w/v), 5% (w/v), and 6% (w/v).

Examples of the preservative that can be blended into the ophthalmic composition of the present invention include invert soaps, parabens, alcohols, and organic acids or salts thereof.

Examples of the invert soaps include benzalkonium chloride, benzalkonium bromide, benzethonium chloride, benzethonium bromide, and chlorhexidine or a salt thereof. When the ophthalmic composition of the present invention contains benzalkonium chloride as the preservative, the blending of the ionic tonicity agent reduces the preservative effectiveness as shown in Examples, described below. Accordingly, when the ophthalmic composition of the present invention contains benzalkonium chloride, it is preferable that the ophthalmic composition contain no ionic tonicity agent. When the ophthalmic composition of the present invention contains benzalkonium chloride, a nonionic tonicity agent is preferably blended as the tonicity agent.

Examples of the parabens include methyl para-oxybenzoate, ethyl para-oxybenzoate, propyl para-oxybenzoate, and butyl para-oxybenzoate.

Examples of the alcohols include chlorobutanol.

Examples of the organic acids or the salts thereof include sorbic acid or a salt thereof. Examples of sorbic acid or a salt thereof include sodium sorbate and potassium sorbate.

When the preservative is blended into the ophthalmic composition of the present invention, the preservative may be used alone or in combination of two or more. The ophthalmic composition of the present invention may not contain any preservative. In this case, it is preferable that the ophthalmic composition contain an ionic tonicity agent as the tonicity agent, and contain no nonionic tonicity agent.

When the preservative is blended into the ophthalmic composition of the present invention, the content of the preservatives can be suitably adjusted depending on the type of the preservative and the like. The content is preferably 0.00001 to 1% (w/v), more preferably 0.0001 to 0.1% (w/v), more preferably 0.0005 to 0.01% (w/v), and the most preferably 0.001 to 0.0075% (w/v). More specifically, for example, the content is preferably 0.001% (w/v), 0.002% (w/v), 0.003% (w/v), 0.004% (w/v), 0.005% (w/v), 0.006% (w/v), 0.007% (w/v), or 0.0075% (w/v).

Examples of the buffer agent that can be blended into the ophthalmic composition of the present invention include phosphoric acid or a salt thereof, boric acid or a salt thereof, citric acid or a salt thereof, acetic acid or a salt thereof, carbonic acid or a salt thereof, tartaric acid or a salt thereof, ε-aminocaproic acid, and trometamol. Among others, phosphoric acid or a salt thereof, and citric acid or a salt thereof are preferable, and citric acid or a salt thereof is more preferable. These include a hydrate thereof.

Examples of phosphoric acid or a salt thereof include phosphoric acid, sodium phosphate, sodium dihydrogen phosphate, disodium hydrogen phosphate, potassium phosphate, potassium dihydrogen phosphate, and dipotassium hydrogen phosphate.

Examples of boric acid or a salt thereof include boric acid, sodium borate, and potassium borate.

Examples of citric acid or a salt thereof include citric acid, sodium citrate, and disodium citrate. Among others, sodium citrate can be preferably used.

Examples of acetic acid or a salt thereof include acetic acid, sodium acetate, and potassium acetate.

Examples of carbonic acid or a salt thereof include sodium carbonate, and sodium hydrogen carbonate.

Examples of tartaric acid or a salt thereof include tartaric acid, sodium tartrate, and potassium tartrate.

When the buffer agent is blended into the ophthalmic composition of the present invention, the buffer agent is used alone or in combination of two or more.

When the buffer agent is blended into the ophthalmic composition of the present invention, the content of the buffer agents can be suitably adjusted depending on the type of the buffer agent and the like, and is preferably 0.001 to 10% (w/v), more preferably 0.01 to 5% (w/v), and further preferably 0.1 to 0.5% (w/v). More specifically, for example, the content is preferably 0.1% (w/v), 0.2% (w/v), 0.3% (w/v), 0.4% (w/v), or 0.5% (w/v).

Examples of the surfactant that can be blended into the ophthalmic composition of the present invention include cationic surfactants, anionic surfactants, and nonionic surfactants. Among others, the nonionic surfactants are preferable.

Examples of the cationic surfactants include alkylamine salts, alkylamine polyoxyethylene adducts, fatty acid triethanolamine monoester salts, acylaminoethyldiethylamine salts, fatty acid polyamine condensates, alkylimidazolines, 1-acylaminoethyl-2-alkylimidazolines, and 1-hydroxylethyl-2-alkylimidazolines.

Examples of the anionic surfactants include phospholipids such as lecithin.

Examples of the nonionic surfactants include polyoxyethylene fatty acid esters such as polyoxyl 40 stearate; polyoxyethylenesorbitan fatty acid esters such as polysorbate 80, polysorbate 60, polysorbate 40, polyoxyethylenesorbitan monolaurate, polyoxyethylenesorbitan trioleate, and polysorbate 65; polyoxyethylene hardened castor oil such as polyoxyethylene hardened castor oil 10, polyoxyethylene hardened castor oil 40, polyoxyethylene hardened castor oil 50, and polyoxyethylene hardened castor oil 60; polyoxyl castor oils such as polyoxyl 5 castor oil, polyoxyl 9 castor oil, polyoxyl 15 castor oil, polyoxyl 35 castor oil, and polyoxyl 40 castor oil; polyoxyethylene polyoxypropylene glycols such as polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, and polyoxyethylene (20) polyoxypropylene (20) glycol; sucrose fatty acid esters such as sucrose stearate ester; and tocopherol polyethylene glycol 1000 succinate ester (vitamin E: TPGS). Among others, polyoxyethylene sorbitan fatty acid esters are preferable, and polysorbate 80 is more preferable.

When the surfactant is blended into the ophthalmic composition of the present invention, the surfactant may be used alone or in combination of two or more.

When the surfactant is blended into the ophthalmic composition of the present invention, the contents of the surfactant can be suitably adjusted depending on the type of the surfactant and the like, and is preferably 0.001 to 10% (w/v), more preferably 0.01 to 5% (w/v), further preferably 0.02 to 1% (w/v), and particularly preferably 0.05 to 0.3% (w/v). More specifically, for example, the content is preferably 0.05% (w/v), 0.1% (w/v), 0.2% (w/v), or 0.3% (w/v).

Examples of the stabilizer that can be blended into the ophthalmic composition of the present invention include sulfites, monoethanolamine, cyclodextrins, dextran, and taurine besides the above-mentioned edetic acid or a salt thereof.

When the stabilizer is blended into the ophthalmic composition of the present invention, the stabilizer is used alone or in combination of two or more.

When a stabilizer is blended into the ophthalmic composition of the present invention besides the above-mentioned edetic acid or a salt thereof, the content of the stabilizer can be suitably adjusted depending on the type of the stabilizer and the like, and is preferably 0.001 to 5% (w/v), more preferably 0.005 to 1% (w/v), and further preferably 0.01 to 0.5% (w/v).

Examples of the antioxidant that can be blended into the ophthalmic composition of the present invention include ascorbic acid, tocopherol, dibutylhydroxytoluene, sodium sulfite, and 2-mercaptobenzimidazole.

When the antioxidant is blended into the ophthalmic composition of the present invention, the antioxidant may be used alone or in combination of two or more.

When the antioxidant is blended into the ophthalmic composition of the present invention, the content of the antioxidants can be suitably adjusted depending on the type of the antioxidant and the like, and is preferably 0.001 to 5% (w/v), more preferably 0.01 to 3% (w/v), and further preferably 0.1 to 2% (w/v).

The pH adjuster that can be blended into the ophthalmic composition of the present invention includes acids and bases. Examples of the acids include hydrochloric acid, phosphoric acid, citric acid, and acetic acid. Examples of the bases include sodium hydroxide, potassium hydroxide, sodium carbonate, and sodium hydrogen carbonate. Suitable addition of these pH adjusters enables adjusting the pH of the ophthalmic composition of the present invention. Specifically, the addition of suitable amounts of hydrochloric acid and sodium hydroxide enables adjusting the pH of the ophthalmic composition.

When the pH adjuster is blended into the ophthalmic composition of the present invention, the pH adjuster may be used alone or in combination of two or more.

When the ophthalmic composition of the present invention is an eye drop, the base is preferably water.

The osmotic pressure ratio of the ophthalmic composition of the present invention only has to be in a range acceptable in an ophthalmic formulation, and is, for example, 0.5 to 2.0, preferably 0.6 to 1.5, more preferably 0.7 to 1.2, and further preferably 0.9 to 1.1.

Based on the Japanese Pharmacopoeia 17th edition, the osmotic pressure ratio is defined as the ratio of the osmotic pressure of a sample to 286 mOsm (the osmotic pressure of 0.9% (w/v) aqueous sodium chloride solution). The osmotic pressure can be measured with reference to the method for measuring the osmotic pressure described in the Japanese Pharmacopoeia (freezing point depression). Sodium chloride (standard reagent prescribed in the Japanese Pharmacopoeia) is dried at 500 to 650°C for 40 to 50 minutes and then cooled in a desiccator (silica gel), 0.900 g thereof is accurately weighed and dissolved in purified water, and the solution is accurately diluted to 100 mL to prepare the standard solution for measuring the osmotic pressure ratio (0.9% (w/v) aqueous sodium chloride solution). Alternatively, a commercial standard solution for measuring the osmotic pressure ratio (0.9% (w/v) aqueous sodium chloride solution) can be used as the standard solution for measuring the osmotic pressure ratio.

The ophthalmic composition of the present invention contains sepetaprost as the active ingredient, and is useful for preventing or treating glaucoma or ocular hypertension or for lowering the intraocular pressure. Examples of glaucoma of the types mentioned in the present invention include primary open-angle glaucoma, secondary open-angle glaucoma, normal tension glaucoma, hypersecretion glaucoma, primary angle-closure glaucoma, secondary angle-closure glaucoma, plateau iris glaucoma, mixed glaucoma, developmental glaucoma, steroid-induced glaucoma, exfoliation glaucoma, amyloid glaucoma, neovascular glaucoma, malignant glaucoma, capsular glaucoma of crystalline lens, and plateau iris syndrome. Preferable examples of glaucoma include primary open-angle glaucoma, normal tension glaucoma, and primary angle-closure glaucoma. The ophthalmic composition of the present invention may contain other active ingredients besides sepetaprost, or may contain sepetaprost alone as the only active ingredient.

The ophthalmic composition of the present invention can be orally or parenterally administered. The administration method may be eye drop administration, intravitreal administration, administration into conjunctival sac, intracameral administration, subconjunctival administration, sub-Tenon's capsule administration, or punctal plug administration. Examples of the dosage form of the ophthalmic composition of the present invention include eye drops, ophthalmic ointment, injections, punctal plugs, tablets, capsules, granules, and powders. Especially eye drops are preferable.

As long as the dosage and administration of the ophthalmic composition of the present invention is sufficient to enable the ophthalmic composition to exhibit desired drug efficacy, the dosage and administration may be any dosage and administration, and can be suitably selected depending on symptoms of disease, the age and the body weight of a patient, the dosage form of the ophthalmic composition, and the like.

Specifically, the eye drop can be ophthalmically administered in a dose of one to five drops, preferably one to three drops, more preferably one to two drops, and particularly preferably one drop; one to four times a day, preferably one to three times a day, more preferably one to two times a day, and particularly preferably once a day; every day to every week. The eye drop is preferably ophthalmically administered in a dose of one drop once a day every day. The one drop as used here is usually around 0.01 to around 0.1 mL, preferably around 0.015 to around 0.07 mL, and more preferably around 0.02 to around 0.05 mL.

The ophthalmic composition of the present invention is preferably a sterilized composition. Examples of the method for sterilizing the ophthalmic composition include election beam (EB) sterilization, ethylene oxide gas (EOG) sterilization, hydrogen peroxide sterilization, gamma ray sterilization, filtration sterilization, and high-pressure steam (AC) sterilization. The method for sterilizing the ophthalmic composition of the present invention is preferably filtration sterilization.

In the present invention, the "election beam sterilization" or "EB sterilization" is a sterilization method that enables killing bacteria and the like by electron beams. As long as the sterilization is a sterilization method using electron beams, the specific method is not particularly limited. For example, the dose of election beam irradiation can be suitably selected from the range of 1 to 100 kGy, and the irradiation time can be suitably selected from the range of several seconds to several minutes.

In the present invention, the "ethylene oxide gas sterilization" or the "EOG sterilization" is a sterilization method that enables killing bacteria and the like with ethylene oxide gas. As long as the sterilization is a sterilization method using ethylene oxide gas, the specific method is not particularly limited. For example, the sterilization temperature may be 30 to 60°C, the sterilization time may be 1 to 10 hours, gas to be used may be ethylene oxide gas alone, or a mixed gas of ethylene oxide gas and carbon dioxide or the like. These can be suitably selected.

In the present invention, the "hydrogen peroxide sterilization" is a sterilization method that enables killing bacteria and the like with hydrogen peroxide. As long as the sterilization is a sterilization method using hydrogen peroxide, the specific method is not particularly limited.

In the present invention, the "gamma ray sterilization" is a sterilization method that enables killing bacteria and the like by gamma ray. As long as the sterilization is a sterilization method using gamma ray, the specific method is not particularly limited.

In the present invention, the "filtration sterilization" is a sterilization method for removing bacteria and the like with a membrane filter (with a pore size of 0.22 µm) by filtration. As the membrane filter, a filter made of a material such as PVDF (polyvinylidene fluoride), PES (polyethersulfone), PTFE (polytetrafluoroethylene), or MCE (cellulose-mixed ester) may be used, and a filter with a different pore size may be used.

In the present invention, the "high-pressure steam (AC) sterilization" is a sterilization method that enables killing bacteria and the like with a high-pressure steam sterilizer (autoclave). As long as the sterilization is a sterilization method using a high-pressure steam sterilizer (autoclave), the specific method is not particularly limited. For example, the high-pressure steam sterilization is performed under the conditions of 115 to 118°C and 30 minutes; 121 to 124°C and 15 to 20 minutes; or 126 to 129°C and 10 minutes, and the sterilization is preferably performed until the target becomes sterilized.

The ophthalmic composition of the present invention can be filled into an airtight container for storage. For example, the ophthalmic composition can be filled into and contained in an eye drop container for storage. Examples of the eye drop container include a resinous (plastic) eye drop container from the viewpoint of material. Examples thereof include a "multi-dose eye drop container" and a "unit dose eye drop container" from the viewpoint of function.

The "multi-dose eye drop container" as used in the present invention means an eye drop container, comprising a container body and a cap attachable to the container body, wherein the cap can be freely opened and resealed. The multi-dose eye drop container usually contains multiple doses of the ophthalmic solution (ophthalmic composition) to be used for a certain period. When the ophthalmic composition of the present invention does not contain a preservative such as benzalkonium chloride, the ophthalmic composition may be contained in a PFMD (preservative-free multi-dose) container. The volume of the ophthalmic composition to be filled into the multi-dose eye drop container or the PFMD container is preferably 1 to 20 mL, more preferably 1 to 15 mL, further preferably 1 to 10 mL, still further preferably 2.5 to 10 mL, and particularly preferably 5 mL.

Meanwhile, the "unit dose eye drop container" means an eye drop container wherein a cap is fusion-sealed on the bottle mouth, which is used by breaking and opening the fusion portion between the cap and the bottle body. The unit dose eye drop container contains one or several doses of the ophthalmic solution (ophthalmic composition). The ophthalmic solution to be filled into the unit dose eye drop container does not commonly contain any preservative such as benzalkonium chloride. For example, the volume of the present composition to be filled into the unit dose eye drop container is preferably 0.1 to 1 mL, more preferably 0.1 to 0.5 mL, further preferably 0.3 mL to 0.5 mL, and particularly preferably 0.3 mL or 0.4 mL.

In the present invention, the material of the multi-dose eye drop container or the unit dose eye drop container is not particularly limited. Various resins (plastics) are usable as the material. Examples of the usable resin include polyethylene (PE), polypropylene (PP), polypropylene-polyethylene copolymer, polyethylene terephthalate (PET), polybutylene terephthalate (PBT), polyvinyl chloride, acrylic resin, and polystyrene. Polyethylene is further classified according to density. Examples of polyethylene include low-density polyethylene (LDPE), medium-density polyethylene (MDPE), and high-density polyethylene (HDPE). In the present invention, polyethylene, polypropylene, propylene-ethylene copolymer, polyethylene terephthalate, and the like are preferably usable among others. As the propylene-ethylene copolymer as mentioned here, any propylene polymer containing an ethylene component can be used, and a propylene polymer containing an ethylene component at 10% by mole or less can be preferably used.

The eye drop container may be formed of one or multiple members, and may be any of a one-piece eye drop container, a two-piece eye drop container, and a three-piece eye drop container. For example, when the eye drop container is a three-piece eye drop container, the eye drop container is formed of three members that are a container body retaining the ophthalmic composition of the present invention, an inner plug, and a cap. The eye drop containers also include integrally molded containers manufactured by blow molding performed simultaneously with filling liquid medicine in accordance with the number of their members. When the container is formed of multiple members, the container may be formed of members made of the same material or different materials. Furthermore, part or the whole of a member may be constituted of, or coated with the material. A container that is commercially available or manufactured by a well-known method is usable.

An aspect of the present invention is a method for stabilizing sepetaprost in an ophthalmic composition, comprising adjusting a pH to a range of 5 to 8 in the ophthalmic composition comprising sepetaprost at a concentration of 0.0001 to 0.003% (w/v). This stabilization method of the present invention is characterized by adjusting a pH to a range of 5 to 8 in the ophthalmic composition comprising sepetaprost at a concentration of 0.0001 to 0.003% (w/v). Detailed description of the above-mentioned ophthalmic composition of the present invention is also applied to this stabilization method.

An aspect of the present invention is a method for stabilizing an ophthalmic composition comprising sepetaprost, characterized by adding edetic acid or a salt thereof at a concentration of more than 0.01% (w/v), and adjusting a pH to a range of 5 to 8. This stabilization method of the present invention is characterized by adding edetic acid or a salt thereof at a concentration of more than 0.01% (w/v) into an ophthalmic composition comprising sepetaprost, and adjusting the pH to a range of 5 to 8. The detailed description of the above-mentioned ophthalmic composition of the present invention is also applied to this stabilization method.

An aspect of the present invention is a method for imparting preservative effectiveness to an ophthalmic composition comprising sepetaprost, characterized by adding the ionic tonicity agent. This method for imparting preservative effectiveness of the present invention is characterized by adding the ionic tonicity agent into an ophthalmic composition comprising sepetaprost. The detailed description of the above-mentioned ophthalmic composition of the present invention is also applied to this method for imparting preservative effectiveness.

An aspect of the present invention is a method for imparting preservative effectiveness to an ophthalmic composition comprising sepetaprost, characterized by adding a nonionic tonicity agent and benzalkonium chloride. This method for imparting preservative effectiveness of the present invention is characterized by adding a nonionic tonicity agent and benzalkonium chloride into an ophthalmic composition comprising sepetaprost. The detailed description of the above-mentioned ophthalmic composition of the present invention is also applied to this method for imparting preservative effectiveness.

An aspect of the present invention is a method for stabilizing sepetaprost in an ophthalmic composition comprising sepetaprost, comprising sterilizing the ophthalmic composition. This stabilization method of the present invention is characterized by comprising sterilizing an ophthalmic composition comprising sepetaprost. The detailed description of the above-mentioned ophthalmic composition of the present invention is also applied to this stabilization method.

An aspect of the present invention is a method for suppressing a decrease in the content of sepetaprost in an ophthalmic composition comprising sepetaprost, comprising filtration-sterilizing the ophthalmic composition. This method for suppressing a decrease in the content of the present invention is characterized by comprising filtration-sterilizing an ophthalmic composition. The detailed description of the above-mentioned ophthalmic composition of the present invention is also applied to the method for suppressing a decrease in the content.

An aspect of the present invention is a method for stabilizing sepetaprost in the ophthalmic composition of the present invention, comprising making the ophthalmic composition contained in a plastic eye drop container. This stabilization method of the present invention is characterized by comprising making the ophthalmic composition of the present invention contained in a plastic eye drop container. The detailed description of the above-mentioned ophthalmic composition of the present invention is also applied to this stabilization method.

An aspect of the present invention is an ophthalmic pharmaceutical product, characterized in that the ophthalmic composition of the present invention is contained in a unit dose eye drop container or a multi-dose eye drop container. This ophthalmic composition of the present invention is characterized in that the ophthalmic composition of the present invention is contained in the unit dose eye drop container or the multi-dose eye drop container. The detailed description of the above-mentioned ophthalmic composition of the present invention is also applied to this ophthalmic pharmaceutical product.

An aspect of the present invention is an ophthalmic pharmaceutical product, characterized in that the ophthalmic composition of the present invention is contained in a plastic eye drop container. This ophthalmic pharmaceutical product of the present invention is characterized in that the ophthalmic composition of the present invention is contained in the plastic eye drop container. The detailed description of the above-mentioned ophthalmic composition of the present invention is also applied to this ophthalmic pharmaceutical product.

An aspect of the present invention is a method for producing an ophthalmic pharmaceutical product, comprising sterilizing the above-mentioned ophthalmic composition of the present invention. This production method of the present invention is characterized by comprising sterilizing the ophthalmic composition of the present invention. The detailed description of the above-mentioned ophthalmic composition of the present invention is also applied to this production method.

### EXAMPLES

Although the present invention will be described in detail hereinafter by giving Test Examples and Formulation Examples, these examples are not for limiting the scope of the present invention in any way but for understanding more about the present invention.

### [Test Example 1]

An influence of pH on the stability of sepetaprost in an ophthalmic composition containing sepetaprost was examined. An influence of sodium edetate on the stability of sepetaprost was also examined.

### (1) Preparation of Test Formulations 1 to 4

Test Formulations 1 to 4 were prepared as shown in Table 1 in accordance with a common method in the art. Specifically, water for injection was added to 0.003 g of sepetaprost, 0.3 g of sodium dihydrogen phosphate dihydrate, and 0.1 g of polysorbate 80, followed by stirring. As a pH adjuster, sodium hydroxide or diluted hydrochloric acid was added in a suitable amount to adjust the pH. A suitable amount of water for injection was added thereto to make a total volume 100 mL, then Test Formulation 1 was prepared. Test Formulations 2 to 4 were also prepared in the same way as Test Formulation 1.

**[Table 1]**

| Composition [% (w/v)] | Test Formulation 1 | Test Formulation 2 | Test Formulation 3 | Test Formulation 4 |
|---|---|---|---|---|
| Sepetaprost | 0.003 | 0.003 | 0.003 | 0.003 |
| Sodium dihydrogen phosphate dihydrate | 0.3 | 0.3 | 0.3 | 0.3 |
| Polysorbate 80 | 0.1 | 0.1 | 0.1 | 0.1 |
| Sodium edetate | - | - | - | 0.12 |
| Sodium hydroxide / diluted hydrochloric acid | q.s. | q.s. | q.s. | q.s. |
| Water for injection | q.s. | q.s. | q.s. | q.s. |
| pH | 4.5 | 6.5 | 8.5 | 6.5 |

### (2) Test method

Test Formulations 1 to 4 were individually filled into glass containers, and these Test Formulations were stored at 70°C for up to 4 weeks. The concentration of sepetaprost in each Test Formulation was measured by high performance liquid chromatography directly before the start of the storage and 1, 2, and 4 weeks after the start of the storage. The residual rate of sepetaprost (%) was calculated based on the concentration of sepetaprost directly before the start of the storage defined as 100%.

### (3) Test results and discussion

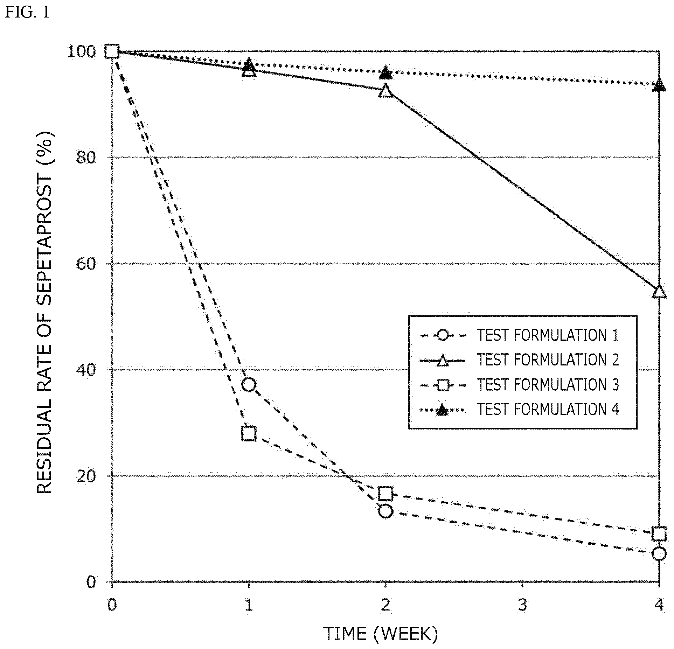

Fig. 1 shows the test results. As shown in Fig. 1, Test Formulations 1 and 3, wherein the pHs were adjusted to 4.5 and 8.5, respectively, markedly decreased in the residual rate of sepetaprost due to the storage at high temperature. Test Formulation 2, wherein the pH was adjusted to 6.5 (no sodium edetate was contained), decreased in the residual rate of sepetaprost from 2 weeks after. Meanwhile, Test Formulation 2 exhibited a high residual rate as compared with Test Formulations 1 and 3. Test Formulation 4, wherein the pH was adjusted to 6.5, and sodium edetate was blended, remained high in the residual rate of sepetaprost even after storage at 70°C for 4 weeks. These results showed that, in an ophthalmic composition containing sepetaprost, the adjustment of the pH to a range of 5 to 8, especially 6.5, enhanced the stability of sepetaprost. The results also showed that blending edetic acid or a salt thereof further enhances the stability of sepetaprost.

### [Test Example 2]

An influence of the concentration of sodium edetate on the stability of sepetaprost in the ophthalmic composition containing sepetaprost was examined.

### (1) Preparation of Test Formulations 5 to 9

Test Formulations 5 to 9 were prepared as shown in Table 2 in accordance with the same method as Test Formulation 1. Test Formulations 5 to 9 were spiked with 0.000001% (w/v) iron nitrate (III).

**[Table 2]**

| Composition [% (w/v)] | Test Formulation 5 | Test Formulation 6 | Test Formulation 7 | Test Formulation 8 | Test Formulation 9 |
|---|---|---|---|---|---|
| Sepetaprost | 0.002 | 0.002 | 0.002 | 0.002 | 0.002 |
| Sodium citrate hydrate | 0.3 | 0.3 | 0.3 | 0.3 | 0.3 |
| Polysorbate 80 | 0.1 | 0.1 | 0.1 | 0.1 | 0.1 |
| Glycerin | 2.3 | 2.3 | 2.3 | 2.3 | 2.3 |
| Sodium edetate | 0 | 0.001 | 0.01 | 0.05 | 0.1 |
| Iron nitrate (III) | 0.000001 | 0.000001 | 0.000001 | 0.000001 | 0.000001 |
| Sodium hydroxide / diluted hydrochloric acid | q.s. | q.s. | q.s. | q.s. | q.s. |
| Water for injection | q.s. | q.s. | q.s. | q.s. | q.s. |
| pH | 6.5 | 6.5 | 6.5 | 6.5 | 6.5 |

### (2) Test method

Test Formulations 5 to 9 were individually filled into polyethylene containers, these Test Formulations was stored at 70°C for 4 weeks. The concentration of sepetaprost, the concentration of the total related substances of sepetaprost, and the concentration of the hydrolysate of sepetaprost in the Test Formulation were measured by high performance liquid chromatography 4 weeks after the start of the storage. The amount of the total related substances (%) and the amount of the hydrolysate (%) were calculated based on the mass of sepetaprost contained in the Test Formulation defined as 100%.

### (3) Test results and discussion

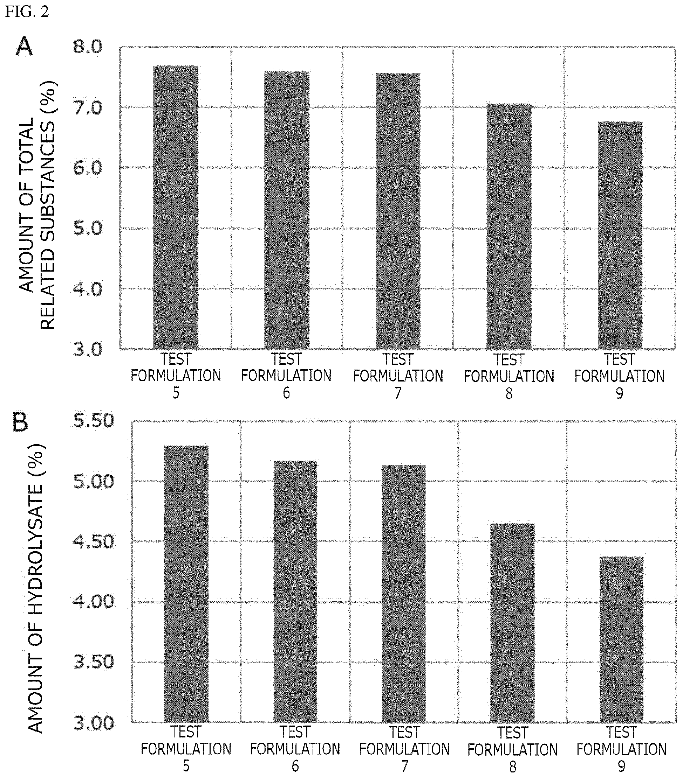

Fig. 2 shows the test results. Fig. 2A is a bar graph showing the amounts of the total related substances (%) of Test Formulations after the storage for 4 weeks. Fig. 2B is a bar graph showing the amounts of the hydrolysate (%) of Test Formulations after the storage for 4 weeks. As shown in Fig. 2, the generation of the related substances and the hydrolysate of sepetaprost was suppressed in Test Formulations 8 and 9 containing sodium edetate at 0.05% (w/v) or more, as compared with the generation in Test Formulation 5 containing no sodium edetate. Especially, the generated amounts of the related substances and the hydrolysate of sepetaprost in Test Formulation 9 containing sodium edetate at 0.1% (w/v) were reduced to around 80%, as compared with those in Test Formulation 5 containing no sodium edetate. These results showed that, in the ophthalmic composition containing sepetaprost, blending sodium edetate at more than 0.01% (w/v), especially at 0.05% (w/v) or more enhances the stability of sepetaprost.

### [Test Example 3]

Various ophthalmic compositions containing sepetaprost were subjected to a preservative effectiveness test.

### (1) Preparation of Test Formulations 10 to 13

Test Formulations 10 to 13 were prepared as shown in Table 3 in accordance with the same method as Test Formulation 1.

**[Table 3]**

| Composition [% (w/v)] | Test Formulation 10 | Test Formulation 11 | Test Formulation 12 | Test Formulation 13 |
|---|---|---|---|---|
| Sepetaprost | 0.003 | 0.003 | 0.003 | 0.003 |
| Sodium citrate hydrate | 0.3 | 0.3 | 0.3 | 0.3 |
| Polysorbate 80 | 0.1 | 0.1 | 0.1 | 0.1 |
| D-Mannitol | 4 | 4 | 4 | 4 |
| Benzalkonium chloride | 0.004 | 0.004 | 0.004 | 0.004 |
| Sodium edetate | 0.06 | 0.096 | 0.096 | 0.096 |
| Sodium hydroxide / diluted hydrochloric acid | q.s. | q.s. | q.s. | q.s. |
| Water for injection | q.s. | q.s. | q.s. | q.s. |
| pH | 6.5 | 6.0 | 6.5 | 7.0 |

### (2) Test method

The preservative effectiveness test was performed in accordance with the Japanese Pharmacopoeia 17th edition and the European Pharmacopoeia 8th edition. In this test, *Escherichia coli (E. coli), Pseudomonas aeruginosa (P. aeruginosa), Staphylococcus aureus* (*S. aureus*), *Candida albicans* (*C*. *albicans*), and *Aspergillus brasiliensis* (*A. brasiliensis*) were used as test bacteria. Bacterial inoculum suspension was inoculated into each test sample, consisting of each Test Formulation, at a bacterial concentration of 10⁵ to 10⁶ cfu/mL in the test sample (for all the five species). Specifically, the bacterial inoculum suspensions were prepared at 10⁷ to 10⁸ cfu/mL, and these bacterial inoculum suspensions were inoculated into the test samples at 10⁵ to 10⁶ cfu/mL, and the mixtures were uniformly mixed to prepare samples. These samples were stored at 20 to 25°C under a light-shielded condition, and aliquots were collected from the samples in an amount of 1 mL at sampling points (6 hours after, 24 hours after, 7 days after, 14 days after, and 28 days after) with a micropippet to measure the numbers of viable bacteria. The lids of the sample solutions were opened for sampling at the sampling points and then closed.

### (3) Test results and discussion

Table 4 shows the test results. The test results in Table 4 are shown as the common logarithm of the ratio (B/A) of the number of bacteria measured at the time of the inoculation (B) to the number of bacteria measured at the time of the sampling (A). For example, when it is indicated as "1", it means that the number of viable bacteria at the time of the sampling has decreased to 10% of the number of bacteria inoculated.

In the preservative effectiveness test, conformance or nonconformance was determined in accordance with criteria of the Japanese Pharmacopoeia 17th edition and the European Pharmacopoeia 8th edition.

**[Table 4]**

| Species | | Test Formulation 10 | Test Formulation 11 | Test Formulation 12 | Test Formulation 13 |
|---|---|---|---|---|---|
| E.coli | 6 hours after | >4.5 | >4.5 | >4.5 | >4.5 |
| | 24 hours after | >4.5 | >4.5 | >4.5 | >4.5 |
| | 7 days after | >4.5 | >4.5 | >4.5 | >4.5 |
| | 14 days after | >4.5 | >4.5 | >4.5 | >4.5 |
| | 28 days after | >4.5 | >4.5 | >4.5 | >4.5 |
| P.aeruginosa | 6 hours after | >4.6 | >4.6 | >4.6 | >4.6 |
| | 24 hours after | >4.6 | >4.6 | >4.6 | >4.6 |
| | 7 days after | >4.6 | >4.6 | >4.6 | >4.6 |
| | 14 days after | >4.6 | >4.6 | >4.6 | >4.6 |
| | 28 days after | >4.6 | >4.6 | >4.6 | >4.6 |
| S.aureus | 6 hours after | >4.6 | 3.4 | >4.6 | 4.6 |
| | 24 hours after | >4.6 | >4.6 | >4.6 | >4.6 |
| | 7 days after | >4.6 | >4.6 | >4.6 | >4.6 |
| | 14 days after | >4.6 | >4.6 | >4.6 | >4.6 |
| | 28 days after | >4.6 | >4.6 | >4.6 | >4.6 |
| C.albicans | 7 days after | >4.5 | >4.5 | >4.5 | >4.5 |
| | 14 days after | >4.5 | >4.5 | >4.5 | >4.5 |
| | 28 days after | >4.5 | >4.5 | >4.5 | >4.5 |
| A.brasiliensis | 7 days after | 2.8 | 2.6 | 2.6 | 2.4 |
| | 14 days after | 3.8 | 3.2 | 3.2 | 3.6 |
| | 28 days after | >4.2 | 4.2 | >4.2 | >4.2 |
| Determination (JP) | | Conformance | Conformance | Conformance | Conformance |
| Determination (EP-A) | | Conformance | Conformance | Conformance | Conformance |

As shown in Table 4, Test Formulations 10 to 13 all conformed to the preservative effectiveness test in accordance with the Japanese Pharmacopoeia and the European Pharmacopoeia, and had sufficient preservative effectiveness.

### [Test Example 4]

An influence of the type of the tonicity agent on the growth of bacteria in the ophthalmic composition containing sepetaprost was examined.

### (1) Preparation of Test Formulations 14 to 16

Test Formulations 14 to 16 were prepared as shown in Table 5 in accordance with the same method as Test Formulation 1.

**[Table 5]**

| Composition [% (w/v)] | Test Formulation 14 | Test Formulation 15 | Test Formulation 16 |
|---|---|---|---|
| Sepetaprost | 0.003 | 0.003 | 0.003 |
| Polysorbate 80 | 0.1 | 0.1 | 0.1 |
| Sodium citrate hydrate | 0.3 | 0.3 | 0.3 |
| Sodium chloride | 0.8 | - | - |
| D-Mannitol | - | 4 | - |
| Glycerin | - | - | 2.3 |
| Sodium hydroxide / diluted hydrochloric acid | q.s. | q.s. | q.s. |
| Water for injection | q.s. | q.s. | q.s. |
| pH | 6.5 | 6.5 | 6.5 |

### (2) Test method

In this test, *Aspergillus brasiliensis* (*A. brasiliensis*) was used as a test bacterium. Test Formulations 14 to 16 were placed in test tubes and used as test samples. The bacterial inoculum suspension was inoculated into each test sample, consisting of each Test Formulation, at a bacterial concentration of 2.5 × 10⁵ cfu/mL in the test sample. Specifically, the bacterial inoculum suspension was prepared at 2.5 × 10⁷ cfu/mL and inoculated into the test samples at 2.5 × 10⁵ cfu/mL, followed by uniform mixing to prepare samples. These samples were stored at 20 to 25°C under the light-shielded condition. Photographs showing the appearances of the test samples 21 days after the seeding of the test bacterium were taken.

### (3) Test results and discussion

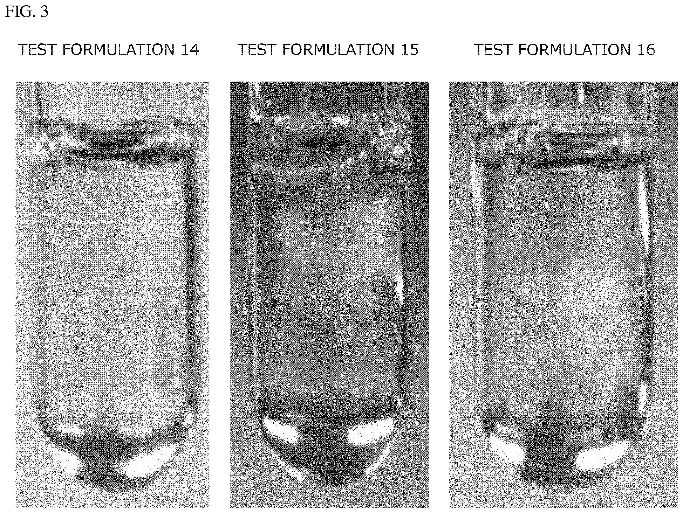

Fig. 3 shows the test results. Fig. 3 is the photographs showing the appearances of the test samples 21 days after the seeding of the test bacterium. As shown in Fig. 3, the appearance of Test Formulation 14 containing sodium chloride as the tonicity agent remained clear even 21 days after the seeding of the test bacterium, and it was not able to be confirmed that the test bacterium (*A*. *brasiliensis*) proliferated therein. In both Test Formulation 15 containing mannitol as the tonicity agent and Test Formulation 16 containing glycerin as the tonicity agent, white floating matter was meanwhile observed in the liquids thereof 21 days after the seeding of the test bacterium, and it was confirmed that the test bacterium (*A*. *brasiliensis*) proliferated. This suggested that the ionic tonicity agent enabled imparting preservative effectiveness to the ophthalmic composition.

### [Test Example 5]

Various ophthalmic compositions containing sepetaprost were subjected to a preservative effectiveness test.

### (1) Preparation of Test Formulations 17 to 23

Test Formulations 17 to 23 were prepared as shown in Table 6 in accordance with the same method as Test Formulation 1.

**[Table 6]**

| Composition [% (w/v)] | Test Formulation 17 | Test Formulation 18 | Test Formulation 19 | Test Formulation 20 | Test Formulation 21 | Test Formulation 22 | Test Formula -tion 23 |
|---|---|---|---|---|---|---|---|
| Sepetaprost | 0.0003 | 0.0003 | 0.0003 | 0.0003 | 0.0003 | 0.0003 | 0.0003 |
| Sodium citrate hydrate | 0.3 | 0.3 | 0.3 | 0.3 | 0.3 | 0.3 | 0.3 |
| Polysorbate 80 | 0.1 | 0.1 | 0.1 | 0.1 | 0.1 | 0.05 | 0.05 |
| Sodium chloride | 0.8 | - | - | - | - | - | - |
| D-Mannitol | - | 4 | 4 | 4 | 4 | 4 | 4 |
| Benzalkonium chloride | 0.005 | 0.0075 | 0.005 | 0.0075 | 0.005 | 0.0075 | 0.005 |
| Sodium edetate | 0.3 | 0.3 | 0.3 | 0.12 | 0.12 | 0.12 | 0.12 |
| Sodium hydroxide / diluted hydrochloric acid | q.s. | q.s. | q.s. | q.s. | q.s. | q.s. | q.s. |
| Water for injection | q.s. | q.s. | q.s. | q.s. | q.s. | q.s. | q.s. |
| pH | 6.5 | 6.5 | 6.5 | 6.5 | 6.5 | 6.5 | 6.5 |
| Osmotic pressure ratio | 1.1 | 1.0 | 1.0 | 0.9 | 0.9 | 0.9 | 0.9 |

### (2) Test method

The preservative effectiveness test was performed in accordance with the Japanese Pharmacopoeia 15th edition, the United States Pharmacopoeia 33rd edition, and the European Pharmacopoeia 7th edition. In this test, *Escherichia coli* (*E. coli*), *Pseudomonas aeruginosa* (*P. aeruginosa*), *Staphylococcus aureus* (*S. aureus*), *Candida albicans* (*C*. *albicans*), and *Aspergillus brasiliensis* (*A. brasiliensis*) were used as test bacteria. Bacterial inoculum suspension was inoculated into each test sample, consisting of each Test Formulation, at a bacterial concentration of 10⁵ to 10⁶ cfu/mL in the test sample (for all the five species). Specifically, the bacterial inoculum suspensions were prepared at 10⁷ to 10⁸ cfu/mL, and these bacterial inoculum suspensions were inoculated into the test samples at 10⁵ to 10⁶ cfu/mL, followed by uniform mixing to prepare samples. These samples were stored at 20 to 25°C under a light-shielded condition, and aliquots were collected from the samples in an amount of 1 mL at sampling points (6 hours after, 24 hours after, 7 days after, 14 days after, and 28 days after) with a micropippet to measure the numbers of viable bacteria. The lids of the sample solutions were opened for sampling at the sampling points and then closed.

### (3) Test results and discussion

Table 7 shows the test results. Table 7 is the results of conformance/nonconformance determination in preservative effectiveness tests in accordance with criteria of the Japanese Pharmacopoeia 15th edition, the United States Pharmacopoeia 33rd edition, and the European Pharmacopoeia 7th edition.

**[Table 7]**

| Determination | Test Formulation 17 | Test Formulation 18 | Test Formulation 19 | Test Formulation 20 | Test Formulation 21 | Test Formulation 22 | Test Formulation 23 |
|---|---|---|---|---|---|---|---|
| JP | Nonconformance | Conformance | Conformance | Conformance | Conformance | Conformance | Conformance |
| USP | Nonconformance | Conformance | Conformance | Conformance | Conformance | Conformance | Conformance |
| EP-A | Nonconformance | Conformance | Conformance | Conformance | Conformance | Conformance | Conformance |
| EP-B | Nonconformance | Conformance | Conformance | Conformance | Conformance | Conformance | Conformance |

As shown in Table 7, all of Test Formulations 18 to 23 conformed to the preservative effectiveness tests in accordance with the Japanese Pharmacopoeia, the United States Pharmacopoeia, and the European Pharmacopoeia, and had sufficient preservative effectiveness. Meanwhile, Test Formulation 17 conformed to none of the preservative effectiveness tests in accordance with the Japanese Pharmacopoeia, the United States Pharmacopoeia, and the European Pharmacopoeia. The comparison between the test results of Test Formulations 17 and 19 showed that while the sample containing benzalkonium chloride as a preservative and the nonionic tonicity agent as the tonicity agent had sufficient preservative effectiveness, the sample containing the ionic tonicity agent instead of the nonionic tonicity agent had insufficient preservative effectiveness. That is, it was revealed that in the case where the ionic tonicity agent was blended in the presence of benzalkonium chloride, the preservative effectiveness was reduced, and that the blending the nonionic tonicity agent enabled maintaining the preservative effectiveness.

The above-mentioned results suggested that the adjustment of the pH to a range of 5 to 8 in the ophthalmic composition containing sepetaprost and the blending of edetic acid or a salt thereof enabled enhancing the stability of sepetaprost. It was suggested that while the blending of an ionic tonicity agent into the ophthalmic composition containing sepetaprost enabled enhancing the preservative effectiveness thereof, the blending of an ionic tonicity agent in the presence of benzalkonium chloride reduced the preservative effectiveness, and the blending of a nonionic tonicity agent enabled maintaining the preservative effectiveness.

### [Test Example 6]

An ophthalmic composition containing sepetaprost was sterilized, and the stability of sepetaprost in the composition was examined.

### (1) Test Formulation

Test Formulation (total volume: 100 mL, pH: 6.5, components: 0.002 g of sepetaprost, 0.3 g of sodium citrate hydrate, 0.1 g of polysorbate 80, 0.75 g of sodium chloride, 0.12 g of sodium edetate hydrate, a suitable amount of a pH adjuster (sodium hydroxide or diluted hydrochloric acid), and a suitable amount of water for injection) was prepared in accordance with a common method in the art.

### (2) Test method

The prepared Test Formulation was subjected to filtration sterilization, or was filled into a unit dose eye drop container or a PFMD container (OSD bottle available from Aptar Group, Inc.), followed by gamma ray sterilization to provide an aseptic formulation. The content of sepetaprost in the Test Formulation subjected to the sterilization processing was quantified by high performance liquid chromatography, and the ratio thereof to the label claim (%) was calculated.

### (3) Results

Table 8 shows the contents of sepetaprost in the Test Formulations subjected to the types of sterilization (the ratio thereof to the label claim %).

**[Table 8]**

| | Sepetaprost content (ratio of content to label claim %) |
|---|---|
| Filtration sterilization | 99.5 |
| Gamma ray sterilization at 25 kGy (unit dose eye drop container) | 5.9 |
| Gamma ray sterilization at 25 kGy (PFMD container) | 6.4 |

These test results confirmed that the formulations subjected to gamma sterilization decreased in the content of sepetaprost. Meanwhile, it was confirmed that in the formulation subjected to the filtration sterilization, a decrease in the content of sepetaprost was effectively suppressed. These results showed that the filtration sterilization of the ophthalmic composition of the present invention enabled maintaining still higher storage stability in the sterilized formulation.

### [Formulation Examples]

Although representative Formulation Examples using the ophthalmic compositions of the present invention will be shown below, the present invention is not limited to only these Formulation Examples. The blended amounts of components in the following Formulation Examples (% (w/v)) are the contents thereof (g) in 100 mL of the ophthalmic compositions.

**[Table 9]**

| Formulation Example | 1 | 2 | 3 | 4 | 5 | 6 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.1 | 0.1 | 0.1 | 0.3 | 0.3 | 0.3 |
| Sodium chloride | 0.75 | 0.75 | 0.75 | 0.75 | 0.75 | 0.75 |
| D-Mannitol | - | - | - | - | - | - |
| Propylene glycol | - | - | - | - | - | - |
| Glycerin | - | - | - | - | - | - |
| Benzalkonium chloride | - | - | - | - | - | - |
| Sodium edetate | 0.05 | 0.05 | 0.05 | 0.05 | 0.05 | 0.05 |
| pH adjuster | q.s. | q.s. | q.s. | q.s. | q.s. | q.s. |
| Water | q.s. | q.s. | q.s. | q.s. | q.s. | q.s. |
| pH | 6.0 | 6.0 | 6.0 | 6.0 | 6.0 | 6.0 |

**[Table 10]**

| Formulation Example | 7 | 8 | 9 | 10 | 11 | 12 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.1 | 0.1 | 0.1 | 0.3 | 0.3 | 0.3 |
| Sodium chloride | 0.75 | 0.75 | 0.75 | 0.75 | 0.75 | 0.75 |
| D-Mannitol | - | - | - | - | - | - |
| Propylene glycol | - | - | - | - | - | - |
| Glycerin | - | - | - | - | - | - |
| Benzalkonium chloride | - | - | - | - | - | - |
| Sodium edetate | 0.12 | 0.12 | 0.12 | 0.12 | 0.12 | 0.12 |
| pH adjuster | q.s. | q.s. | q.s. | q.s. | q.s. | q.s. |
| Water | q.s. | q.s. | q.s. | q.s. | q.s. | q.s. |
| pH | 6.5 | 6.5 | 6.5 | 6.5 | 6.5 | 6.5 |

**[Table 11]**

| Formulation Example | 13 | 14 | 15 | 16 | 17 | 18 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.1 | 0.1 | 0.1 | 0.3 | 0.3 | 0.3 |
| Sodium chloride | 0.1 | 0.1 | 0.1 | 0.1 | 0.1 | 0.1 |
| D-Mannitol | - | - | - | - | - | - |
| Propylene glycol | - | - | - | - | - | - |
| Glycerin | - | - | - | - | - | - |
| Benzalkonium chloride | - | - | - | - | - | |
| Sodium edetate | 0.12 | 0.12 | 0.12 | 0.12 | 0.12 | 0.12 |
| pH adjuster | q.s. | q.s. | q.s. | q.s. | q.s. | q.s. |
| Water | q.s. | q.s. | q.s. | q.s. | q.s. | q.s. |
| pH | 7.0 | 7.0 | 7.0 | 7.0 | 7.0 | 7.0 |

**[Table 12]**

| Formulation Example | 19 | 20 | 21 | 22 | 23 | 24 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.3 | 0.3 | 0.3 | 0.1 | 0.1 | 0.1 |
| Sodium chloride | - | - | - | - | - | - |
| D-Mannitol | 4 | 4 | 4 | 4 | 4 | 4 |
| Propylene glycol | - | - | - | - | - | - |
| Glycerin | - | - | - | - | - | - |
| Benzalkonium chloride | 0.005 | 0.005 | 0.005 | 0.005 | 0.005 | 0.005 |
| Sodium edetate | 0.12 | 0.12 | 0.12 | 0.12 | 0.12 | 0.12 |
| pH adjuster | q.s. | q.s. | q.s. | q.s. | q.s. | q.s. |
| Water | q.s. | q.s. | q.s. | q.s. | q.s. | q.s. |
| pH | 6.5 | 6.5 | 6.5 | 6.5 | 6.5 | 6.5 |

**[Table 13]**

| Formulation Example | 25 | 26 | 27 | 28 | 29 | 30 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.3 | 0.3 | 0.3 | 0.1 | 0.1 | 0.1 |
| Sodium chloride | - | - | - | - | - | - |
| D-Mannitol | 2 | 2 | 2 | 2 | 2 | 2 |
| Propylene glycol | - | - | - | - | - | - |
| Glycerin | - | - | - | - | - | - |
| Benzalkonium chloride | 0.005 | 0.005 | 0.005 | 0.005 | 0.005 | 0.005 |
| Sodium edetate | 0.05 | 0.05 | 0.05 | 0.05 | 0.05 | 0.05 |
| pH adjuster | q.s. | q.s. | q.s. | q.s. | q.s. | q.s. |
| Water | q.s. | q.s. | q.s. | q.s. | q.s. | q.s. |
| pH | 6.0 | 6.0 | 6.0 | 6.0 | 6.0 | 6.0 |

**[Table 14]**

| Formulation Example | 31 | 32 | 33 | 34 | 35 | 36 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.3 | 0.3 | 0.3 | 0.1 | 0.1 | 0.1 |
| Sodium chloride | - | - | - | - | - | - |
| D-Mannitol | | | | | | |
| Propylene glycol | 2 | 2 | 2 | 2 | 2 | 2 |
| Glycerin | - | - | - | - | - | - |
| Benzalkonium chloride | 0.005 | 0.005 | 0.005 | 0.005 | 0.005 | 0.005 |
| Sodium edetate | 0.05 | 0.05 | 0.05 | 0.05 | 0.05 | 0.05 |
| pH adjuster | q.s. | q.s. | q.s. | q.s. | q.s. | q.s. |
| Water | q.s. | q.s. | q.s. | q.s. | q.s. | q.s. |
| pH | 6.5 | 6.5 | 6.5 | 6.5 | 6.5 | 6.5 |

**[Table 15]**

| Formulation Example | 37 | 38 | 39 | 40 | 41 | 42 |
|---|---|---|---|---|---|---|
| Sepetaprost | 0.002 | 0.002 | 0.002 | 0.002 | 0.002 | 0.002 |
| Polysorbate 80 | 0.05 | 0.1 | 0.2 | 0.05 | 0.1 | 0.2 |
| Sodium citrate hydrate | 0.3 | 0.3 | 0.3 | 0.1 | 0.1 | 0.1 |
| Sodium chloride | - | - | - | - | - | - |
| D-Mannitol | - | - | - | - | - | - |
| Propylene glycol | - | - | - | - | - | - |
| Glycerin | 2 | 2 | 2 | 2 | 2 | 2 |
| Benzalkonium chloride | 0.005 | 0.005 | 0.005 | 0.005 | 0.005 | 0.005 |
| Sodium edetate | 0.05 | 0.05 | 0.05 | 0.05 | 0.05 | 0.05 |
| pH adjuster | q.s. | q.s. | q.s. | q.s. | q.s. | q.s. |
| Water | q.s. | q.s. | q.s. | q.s. | q.s. | q.s. |
| pH | 6.0 | 6.0 | 6.0 | 6.0 | 6.0 | 6.0 |

### INDUSTRIAL APPLICABILITY

The present invention enables enhancing the stability of sepetaprost in an ophthalmic composition and providing an ophthalmic composition wherein its stability can be maintained even on exposure to high temperatures in the distribution process or the storage process. Additively, the present invention enables providing an ophthalmic composition wherein the preservative effectiveness is maintained by the use of a suitable type of tonicity agent, in addition to maintaining the stability of sepetaprost. Furthermore, the present invention enables providing an ophthalmic composition wherein high stability of sepetaprost in the ophthalmic composition is maintained by the selection of a method for sterilizing the ophthalmic composition containing sepetaprost.

## Claims

1. An ophthalmic composition comprising sepetaprost at a concentration of 0.0001 to 0.003% (w/v), wherein the pH of the composition is in a range of 5 to 8.

2. The ophthalmic composition according to claim 1, further comprising edetic acid or a salt thereof at a concentration of more than 0.01% (w/v).

3. The ophthalmic composition according to claim 2, wherein the concentration of edetic acid or a salt thereof is 0.05% (w/v) or more.

4. The ophthalmic composition according to claim 2, wherein the concentration of edetic acid or a salt thereof is 0.05 to 0.5% (w/v).

5. The ophthalmic composition according to claim 2, wherein the concentration of edetic acid or a salt thereof is 0.05 to 0.2% (w/v).

6. The ophthalmic composition according to any one of claims 1 to 5, wherein the pH is in the range of 6 to 7.

7. The ophthalmic composition according to any one of claims 1 to 5, wherein the pH is 6.5.

8. The ophthalmic composition according to any one of claims 1 to 7, further comprising a tonicity agent.

9. The ophthalmic composition according to claim 8, wherein the tonicity agent is an ionic tonicity agent.

10. The ophthalmic composition according to claim 9, comprising no benzalkonium chloride.

11. The ophthalmic composition according to claims 9, comprising no preservative.

12. The ophthalmic composition according to any one of claims 9 to 11, comprising no nonionic tonicity agent.

13. The ophthalmic composition according to claim 8, wherein the tonicity agent is a nonionic tonicity agent.

14. The ophthalmic composition according to claim 13, further comprising benzalkonium chloride.

15. The ophthalmic composition according to claim 13 or 14, comprising no ionic tonicity agent.

16. An ophthalmic composition comprising sepetaprost at a concentration of 0.0001 to 0.003% (w/v); a nonionic tonicity agent; and benzalkonium chloride, wherein the pH of the composition is in a range of 5 to 8.

17. The ophthalmic composition according to claim 16, comprising no ionic tonicity agent.

18. An ophthalmic composition comprising sepetaprost at a concentration of 0.0001 to 0.003% (w/v); an ionic tonicity agent; and no benzalkonium chloride, wherein the pH of the composition is in a range of 5 to 8.

19. The ophthalmic composition according to claim 18, comprising no preservative.

20. An ophthalmic composition comprising:
sepetaprost at a concentration of 0.0001 to 0.003% (w/v);
edetic acid or a salt thereof at a concentration of more than 0.01% (w/v);
an ionic tonicity agent;
no nonionic tonicity agent; and
no benzalkonium chloride,
wherein the pH of the composition is in a range of 5 to 8.

21. An ophthalmic composition comprising:
sepetaprost at a concentration of 0.0001 to 0.003% (w/v);
edetic acid or a salt thereof at a concentration of more than 0.01% (w/v);
a nonionic tonicity agent;
benzalkonium chloride; and
no ionic tonicity agent,
wherein the pH of the composition is in a range of 5 to 8.

22. The ophthalmic composition according to any one of claims 12 to 17, 20, and 21, wherein the nonionic tonicity agent is at least one selected from the group consisting of mannitol, glycerin, propylene glycol, polyethylene glycol, sorbitol, trehalose, maltose, and sucrose.

23. The ophthalmic composition according to claim 22, wherein the nonionic tonicity agent is mannitol.

24. The ophthalmic composition according to any one of claims 9 to 12, 15, and 17 to 23, wherein the ionic tonicity agent is at least one selected from the group consisting of sodium chloride, potassium chloride, calcium chloride, and magnesium chloride.

25. The ophthalmic composition according to claim 24, wherein the ionic tonicity agent is sodium chloride.

26. The ophthalmic composition according to any one of claims 1 to 25, further comprising a buffer agent.

27. The ophthalmic composition according to any one of claims 1 to 26, further comprising a surfactant.

28. The ophthalmic composition according to any one of claims 1 to 27, wherein the ophthalmic composition is a sterilized composition.

29. The ophthalmic composition according to claim 28, wherein the sterilization is filtration sterilization.

30. An ophthalmic composition comprising:
sepetaprost at a concentration of 0.0001 to 0.003% (w/v);
polysorbate 80 at a concentration of 0.05 to 0.3% (w/v);
sodium citrate hydrate at a concentration of 0.1 to 0.5% (w/v);
sodium edetate at a concentration of 0.05 to 0.5% (w/v);
sodium chloride at a concentration of 0.5 to 1% (w/v); and
no benzalkonium chloride,
wherein the pH of the composition is 6 to 7.

31. An ophthalmic composition comprising:
sepetaprost at a concentration of 0.0001 to 0.003% (w/v);
polysorbate 80 at a concentration of 0.05 to 0.3% (w/v);
sodium citrate hydrate at a concentration of 0.1 to 0.5% (w/v);
sodium edetate at a concentration of 0.05 to 0.5% (w/v);
mannitol at a concentration of 1 to 6% (w/v); and
benzalkonium chloride at a concentration of 0.001 to 0.0075% (w/v),
wherein the pH of the composition is 6 to 7.

32. An ophthalmic composition comprising:
sepetaprost at a concentration of 0.0001 to 0.003% (w/v);
polysorbate 80 at a concentration of 0.05 to 0.3% (w/v);
sodium citrate hydrate at a concentration of 0.1 to 0.5% (w/v);
sodium edetate at a concentration of 0.05 to 0.5% (w/v);
sodium chloride at a concentration of 0.5 to 1% (w/v); and
no benzalkonium chloride,
wherein the pH of the composition is 6.5.

33. An ophthalmic composition comprising:
sepetaprost at a concentration of 0.0001 to 0.003% (w/v);
polysorbate 80 at a concentration of 0.05 to 0.3% (w/v);
sodium citrate hydrate at a concentration of 0.1 to 0.5% (w/v);
sodium edetate at a concentration of 0.05 to 0.5% (w/v);
mannitol at a concentration of 1 to 6% (w/v); and
benzalkonium chloride at a concentration of 0.001 to 0.0075% (w/v),
wherein the pH of the composition is 6.5.

34. The ophthalmic composition according to any one of claims 1 to 33, wherein the concentration of sepetaprost is 0.001 to 0.003% (w/v).

35. The ophthalmic composition according to any one of claims 1 to 33, wherein the concentration of sepetaprost is 0.002 to 0.003% (w/v).

36. The ophthalmic composition according to any one of claims 1 to 33, wherein the concentration of sepetaprost is 0.002% (w/v).

37. An ophthalmic composition comprising sepetaprost at a concentration of 0.002 to 0.003% (w/v), wherein the pH of the composition is in a range of 6 to 7.

38. An ophthalmic composition comprising sepetaprost at a concentration of 0.002% (w/v), wherein the pH of the composition is in a range of 6 to 7.

39. The ophthalmic composition according to any one of claims 1 to 38, wherein the ophthalmic composition is an eye drop.

40. The ophthalmic composition according to any one of claims 1 to 39, wherein the ophthalmic composition is contained in a unit dose eye drop container.

41. The ophthalmic composition according to any one of claims 1 to 39, wherein the ophthalmic composition is contained in a multi-dose eye drop container.

42. The ophthalmic composition according to any one of claims 1 to 41, wherein the ophthalmic composition is contained in a plastic eye drop container.

43. The ophthalmic composition according to any one of claims 1 to 42, wherein the ophthalmic composition is for preventing or treating glaucoma or ocular hypertension.

44. A method for stabilizing sepetaprost in an ophthalmic composition, comprising adjusting a pH to a range of 5 to 8 in the ophthalmic composition comprising sepetaprost at a concentration of 0.0001 to 0.003% (w/v).

45. The method according to claim 44, wherein the pH is adjusted to the range of 6 to 7 in the ophthalmic composition comprising sepetaprost at a concentration of 0.002 to 0.003% (w/v).

46. The method according to claim 44 or 45, wherein the pH is adjusted to the range of 6 to 7 in the ophthalmic composition comprising sepetaprost at a concentration of 0.002% (w/v).
